(19) Europäisches Patentamt
European Patent Office
Office européen des brevets

(11) Veröffentlichungsnummer: **0 232 500 A2**

# (12) EUROPÄISCHE PATENTANMELDUNG

(21) Anmeldenummer: **86116834.2**

(22) Anmeldetag: **03.12.86**

(51) Int. Cl.⁴: **C 07 K 5/02**, C 07 K 5/06, C 07 K 5/08, A 01 N 57/20, A 01 N 57/22

(30) Priorität: **14.12.85 DE 3544375**

(43) Veröffentlichungstag der Anmeldung: **19.08.87 Patentblatt 87/34**

(84) Benannte Vertragsstaaten: **AT BE CH DE FR GB IT LI NL**

(71) Anmelder: **HOECHST AKTIENGESELLSCHAFT, Postfach 80 03 20, D-6230 Frankfurt am Main 80 (DE)**

(72) Erfinder: **Kehne, Heinz, Dr., Berliner Strasse 10, D-6238 Hofheim am Taunus (DE)**
Erfinder: **Mildenberger, Hilmar, Dr., Fasanenstrasse 24, D-6233 Kelkheim (Taunus) (DE)**
Erfinder: **Bauer, Klaus, Dr., Kolpingstrasse 7, D-6054 Rodgau (DE)**
Erfinder: **Bieringer, Hermann, Dr., Eichenweg 26, D-6239 Eppstein/Taunus (DE)**

(54) **Di- und Tripeptide mit N-terminalem Phosphinothricin, Verfahren zu ihrer Herstellung und ihre Verwendung zur Bekämpfung unerwünschten Pflanzenwuchses.**

(57) Di- und Tripeptide mit N-terminalem Phosphinothricin der Formel

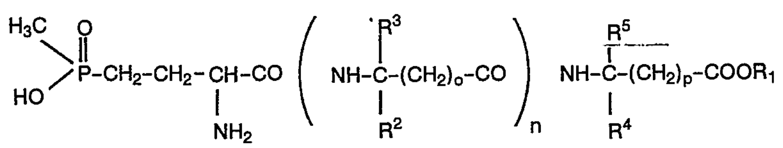

(I)

in denen Phosphinothricin mit 1 oder 2 Molekülen von vorzugsweise natürlichen Aminosäuren verknüpft ist, sind wirksame Herbizide im Vor- und Nachauflauf. Man erhält die Verbindungen durch Umsetzung von an der Aminogruppe geschützten Phosphinothricin mit – an der terminalen Carboxylgruppe veresterten – Aminosäuren oder Dipeptiden in Gegenwart einer Base und eines Carbodiimids und ggf. anschließende Abspaltung der Schutzgruppen.

EP 0 232 500 A2

## Di- und Tripeptide mit N-terminalem Phosphinothricin, Verfahren zu ihrer Herstellung und ihre Verwendung zur Bekämpfung unerwünschten Pflanzenwuchses

Es ist bekannt, daß das Phosphinothricin (Ptc)

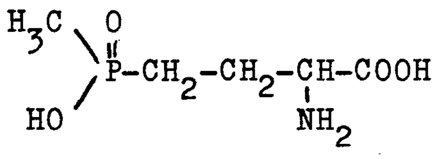

und einige seiner Peptide gute herbizide Wirksamkeit aufweisen. Von den letzteren sind bereits beschrieben: H-Ptc-Gly-OH und H-Ptc-Ala-OH (JA-Sho-55-7237); H-Ptc-Ala-Ala-OH (Bialaphos oder SF-1293; DE-AS 2 236 599 und DE-PS 2 848 224), H-Ptc-Ala-Leu-OH (J. Antibiotic. 37, 829 (1984)) und einer Reihe weiterer N-terminaler Ptc-Tripeptide (JA-A Sho-60-222498). Eine Anzahl von Dipeptiden mit N-terminalem Ptc wird außerdem formal vom Anspruch der DE-PS 27 17 440 umfaßt. In der Patentschrift werden jedoch weder Beispiele für Dipeptide erwähnt noch eine Methode zu ihrer Herstellung angegeben. Die bisher bekannt gewordenen Synthesemethoden sind langwierig und bisher nur in kleinem Maßstab durchgeführt worden. Dies liegt an der Phosphinylgruppe des Ptc, die bei den herkömmlichen Verfahren geschützt werden muß, da sonst Nebenreaktionen auftreten, die die Ausbeute und Reinheit der Endprodukte stark beeinträchtigen.

Ziel der vorliegenden Erfindung ist die Bereitstellung neuer, herbizid wirksamer Di- und Tripeptide mit N-terminalem Ptc und die Angabe eines vergleichsweise einfachen Synthesewegs.

Gegenstand der Erfindung sind somit Verbindungen der allgemeinen Formel I,

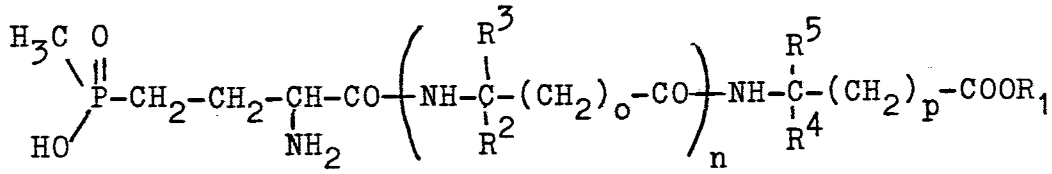 (I)

worin

$R^1$ Wasserstoff oder $(C_1-C_{12})$-Alkyl,

$R^2$ und $R^4$ unabhängig voneinander Wasserstoff, $(C_1-C_{12})$-Alkyl, $(C_3-C_8)$-Cycloalkyl, Phenyl oder Phenyl$(C_1-C_2)$-alkyl, wobei die genannten Gruppen ihrerseits durch Halogen, $NH_2$, OH, SH, $NO_2$, $CF_3$ $(C_1-C_4)$-Alkoxy, $(C_1-C_4)$-Alkylthio, $(C_1-C_4)$-Alkoxycarbonyl, COOH oder $CONH_2$ substituiert sein können,

$R^3$ und $R^5$ Wasserstoff oder Methyl,

n 0 oder 1 und

o und p unabhängig voneinander 0, 1, 2 oder 3 bedeuten,

ausgenommen Verbindungen, in denen

a) n = 0, $R^4$= H oder $CH_3$ und $R^5$= H; oder

b) n = 1, $R^3$ und $R^5$= H sowie $R^2$ und $R^4$= H, $CH_3$, $C_2H_5$, $CH(CH_3)_2$ oder $CH_2OH$; oder

c) n = 1, $R^3$ und $R^5$= H, $R^2$= $CH_3$ und $R^4$= $CH_2CH(CH_3)_2$ bedeuten,

sowie deren Salze mit Basen bzw. Säuren.

Die Verbindungen der Formel I enthalten je nach Substitution 1, 2 oder 3 asymmetrische Kohlenstoffatome. Sie können also in mehreren stereoisomeren Formen auftreten. Die Erfindung betrifft daher nicht nur die Racemate der Formel I, sondern auch deren Stereoisomere und Gemische derselben in beliebigen Verhältnissen.

Bevorzugte Verbindungen sind solche, in denen

$R^1$ Wasserstoff oder $(C_1-C_8)$-Alkyl,

$R^2$ und $R^4$ unabhängig voneinander H, $(C_1-C_8)$Alkyl, $(C_3-C_4)$-Aminoalkyl, Hydroxymethyl, Hydroxyethyl, Thiomethyl, Methylthioethyl, Carboxymethyl oder -ethyl, Carbamoylmethyl oder -ethyl, Carbo$(C_1-C_4)$-alkoxymethyl oder -ethyl, Phenyl, Benzyl oder Hydroxybenzyl,

$R^3$ und $R^5$ H oder $CH_3$,

n 0 oder 1, und

o und p unabhängig voneinander 0, 1 oder 2 bedeuten, (ausgenommen die vorstehend unter a), b) und c) aufgeführten Verbindungen) sowie deren Salze.

Besonders bevorzugt sind solche Verbindungen, in denen Ptc mit "natürlichen", d.h. proteinogenen Aminosäuren wie Alanin (Ala), α-Aminobuttersäure (Abu), Asparagin (Asn), Asparaginsäure (Asp), Cystein (Cys), Glutamin (Gln), Glutaminsäure (Glu), Glycin (Gly), Isoleucin (Ile), Leucin (Leu), Lysin (Lys), Methionin (Met), Ornithin (Orn), Phenylalanin (Phe), Serin (Ser), Threonin (Thr), Tyrosin (Tyr), Valin (Val) oder auch nicht-proteinogenen Aminosäuren wie Phenylglycin (Phg) oder Norvalin (Nva) ein- oder zweifach N-terminal verknüpft ist.

Da die Verbindungen der Formel I saure (-COOH, -P-(O)OH) und basische ($-NH_2$) Gruppen enthalten, sind sie zu Salzbildung mit Basen und starken Säuren befähigt. Als Salze mit Basen kommen insbesondere die Alkali- und Erdalkalisalze (mit $Na^+$, $K^+$ oder $Ca^{++}$), Ammoniumsalze sowie Salze mit organischen Ammoniumbasen wie Mono-, Di und Tri-alkyl-($C_1$-$C_4$)- bzw. -hydroxyethylammoniumsalze, ferner die Benzyl-, Dibenzyl-, Phenyl-, Diphenyl- und Dicyclohexylammoniumsalze in Betracht. Zur Salzbildung an der Aminogruppe eignen sich besonders starke Säuren wie HCl, HBr, $H_2SO_4$, $HNO_3$, $H_3PO_4$, $HClO_4$ und Benzolsulfonsäure.

Gegenstand der Erfindung ist auch ein Verfahren zur Herstellung der Verbindungen der allgemeinen Formel I, das dadurch gekennzeichnet ist, daß man eine Verbindung der Formel II,

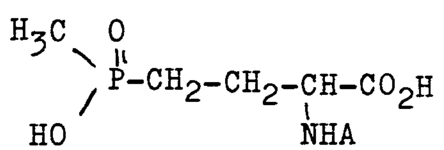

(II)

worin A eine Amino-Schutzgruppe bedeutet, mit dem Salz eines Aminosäure- bzw. Dipeptidderivats der Formel III,

$$H_2N\left(\begin{matrix}R^3\\|\\C-CONH\\|\\R^2\end{matrix}\right)_n\begin{matrix}R^5\\|\\C-COOR^6\\|\\R^4\end{matrix}\qquad (III)$$

worin $R_6$ eine in der Peptidchemie gebräuchliche Carboxyl-Schutzgruppe wie Niederalkyl oder Benzyl ist, in Gegenwart einer Base und eines Dialkylcarbodiimids wie Dicyclohexylcarbodiimid (DCC) als Kondensationsmittel umsetzt und anschließend, soweit gewünscht, die vorhandenen Schutzgruppen abspaltet.

Als Komponenten III kommen vor allem die Salze der natürlichen Aminosäuren wegen ihrer leichten Zugänglichkeit in Betracht. Soweit es sich bei den Ausgangsstoffen nicht um handelsübliche Substanzen handelt, sind sie nach literaturbekannten Methoden darstellbar. Als Salze von III kommen insbesondere die Hydrochloride, aber auch Salze mit anderen starken Säuren in Frage. Die Schutzgruppen an II sind die in der Peptidchemie üblichen; beispielsweise seien die Carbobenzoxy (Cbo-)gruppe und tert. Butyloxycarbonyl (Boc-) gruppe genannt. Die Kondensation der beiden Komponenten führt man zweckmäßigerweise in einem inerten Lösungsmittel wie $CH_2Cl_2$, Dioxan, Tetrahydrofuran, Essigsäureethylester, Dimethoxyethan, Acetonitril oder Dimethylformamid bei Temperaturen zwischen -20°C und 60°C, bevorzugt zwischen -10°C und 50°C in Gegenwart einer Hilfsbase und eines Dialkylcarbodiimids, z.B. Dicyclohexylcarbodiimid, aus. Als Hilfsbasen eignen sich tertiäre organische Amine wie Triethylamin, N-Ethylmorpholin oder Pyridin.

Nach der Peptidverknüpfung verbleiben Schutzgruppen im Molekül, die nach literaturbekannten Methoden abgespalten werden können, z.B. die Cbo-Gruppe und die Benzylgruppe durch Hydrogenolyse, die Boc-Gruppe durch Acidolyse, Alkylestergruppen durch saure oder alkalische Hydrolyse.

Je nach Art und Reihenfolge der Schutzgruppenabspaltung erhält man die erfindungsgemäßen Verbindungen I in freier Form oder als Salze. Die Salze können nach literaturbekannten Methoden (z.B. durch Umsetzung der Hydrochloride mit Propylenoxid) in die freien Peptide übergeführt werden. Aus diesen erhält man Salze durch Titration mit einem Äquivalent Säure bzw. Base und Eindampfen der wäßrigen Lösung.

Ein besonderer Vorteil des erfindungsgemäßen Herstellungsverfahrens besteht darin, daß man auf eine Maskierung der Phosphinsäurefunktion verzichten kann. Obwohl letztere durchaus zu Reaktionen mit dem Kondensationsmittel befähigt wäre, verläuft die Knüpfung der Peptidverbindung einheitlich über die Carobxylfunktion.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Die Verbindungen der Formel I können mit zahlreichen anderen Herbiziden kombiniert werden; die Mischungen besitzen teilweise synergistische Wirkung. Beispiele für solche Mischungspartner sind:

Triazine wie 2-Chlor-4-ethylamino-6-isopropylamino-1,3,5-triazin (Atrazin), 2-Chlor-4,6-bis(ethylamino)-1,3,5-triazin (Simazin), 2-tert.-Butylamino-4-chlor-6-ethylamino-1,3,5-triazin (Terbutylazine), 2-tert.-Butylamino-4-ethylamino-6-methoxy-1,3,5-triazin (Terbumeton), 2-Ethylamino-4-isopropylamino-6-methylthio-1,3,5-triazin (Ametryne), 2,4-Bis(isopropylamino)-6-methylthio-1,3,5-triazin (Prometryn) oder 2-tert.-Butylamino-4-ethylamino-6-methylthio-1,3,5-triazin (Terbutryne),

Wuchsstoffe wie 2,4-Dichlorphenoxyessigsäure (2,4-D), 4-Chlor-2-methylphenoxyessigsäure (MCPA), 4-Chlor-2-methylphenoxypropionsäure (CMPP), 2-(2,4-Dichlorphenoxy)-propionsäure (2,4-DP), 2,4,5-Trichlorphenoxyessigsäure (2,4,5-T), 3,6-Dichlor-2-methoxybenzoesäure (Dicamba) und deren Ester und Salze,

Acetanilide wie 2-Chlor-2',6'-diethyl-N-methoxymethylacetanilid Alachlor , 2-Chlor-6'-ethyl-N-(2-methoxy-1-methylethyl)-acet-o-toluidid Metolachlor,oder 2-Chlor-N-(ethoxymethyl)-6'-ethylacet -o-toluidid,

Phenylharnstoffe wie 3-(3,4-Dichlorphenyl)-1-methoxy-1-methylharnstoff (Linuron), 3-(3,4-Dichlorphenyl)-1,1-dimethylharnstoff (Diuron), 3-(4-Chlorphenyl)-1-methoxy-1-methylharnstoff (Monolinuron),

ferner 2-[3-(4,6-Dimethylpyrimidin-2-yl)ureidosulfonyl]-benzoesäuremethylester, 2-(4-Isopropyl-4-methyl-5-oxo-2-imidazolin-2-yl)-nicotinsäure-isopropylammoniumsalz, 2-(4-Isopropyl)-4-methyl-5-oxo-2-imidazolin-2-yl)chinolin-3-carbonsäure, 2-[3-(4-Chlor-6-methoxypyrimidin-2-yl)-ureidosulfonyl]-benzoesäuremethylester, 4-Amino-6-tert.-butyl-4,5-dihydro-3-methylthio-1,2,4-triazin-5-on, 3-Amino-1H-1,2,4-triazol, 2,6-Dinitro-N,N-dipropyl-4-trifluormethylanilin (Trifluralin), Ammoniumethylcarbamoylphosphonat und N-(Phosphonomethyl)glycin (Glyphosate).

Bei Applikation der Wirkstoffe auf die grünen Pflanzenteile im Nachauflaufverfahren tritt sehr rasch nach der Behandlung ein drastischer Wachstumsstop ein, und die Unkrautpflanzen bleiben in dem zum Applikationszeitpunkt vorhandenen Wachstumsstadium stehen und sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Die erfindungsgemäßen Verbindungen besitzen auch eine ausgezeichnete und sehr breite Wirkung gegen ein weites Spektrum annueller sowie perennierender Ungräser und Unkräuter, die an Wegrändern, in Industrie- oder Eisenbahnanlagen wachsen. Die Wirkstoffe eignen sich deshalb sowohl zur Anwendung auf nicht landwirtschaftlich genutzten Flächen als auch zur Unkrautbekämpfung in der Landwirtschaft. Der Einsatz von nichtselektiven Verbindungen in ein oder mehrjährigen landwirtschaftlichen Kulturen ist möglich, sofern durch die Art der Applikation und/oder das Alter der Kulturpflanzen sichergestellt wird, daß die Kulturpflanzen bzw. ihre empfindlichen grünen Teile nicht besprüht werden und so keinen Schaden leiden. Beispiele für derartige Einsatzmöglichkeiten sind Plantagen, Baumkulturen, Rebanlagen etc..

Da eine Anwendung der neuen Verbindungen den einjährigen Nutzkulturen vor dem Auflaufen der Kulturpflanzen oder in deren Reifestadium keinen Schaden zufügt, kann man sie auch vor der Aussaat, kurz vor oder nach der Ernte im Zuge der minimalen Bodenbearbeitung, der Bekämpfung von Spätverunkrautung und der Ernteerleichterung einsetzen.

Gegenstand der Erfindung sind daher auch herbizide Mittel, die die Verbindung der Formel I enthalten, und deren Verwendung zur Bekämpfung von unerwünschtem Pflanzenwuchs.

./.

Die erfindungsgemäßen Mittel enthalten die Wirkstoffe gemäß der allgemeinen Formel I zu 2-95%. Da die Wirkstoffe zum Teil wasserlöslich sind, können sie vorteilhaft als wäßrige Lösungen eingesetzt werden. Andernfalls können sie als emulgierbare Konzentrate, benetzbare Pulver und versprühbare Lösungen in den üblichen Zubereitungsformen angewendet werden, sofern sie nicht selbst wasserlöslich sind.

Benetzbare Pulver sind in Wasser gleichmäßig dispergierbare Präparate, die neben dem Wirkstoff außer einem Verdünnungs- oder Inertstoff noch Netzmittel, z.B. polyoxäthylierte Alkylphenole, polyoxäthylierte Oleyl- oder Stearylamine, Alkyl- oder Alkylphenyl-sulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z. B. Butanol, Cyclohexanon, Xylol oder auch höhersiedenden Aromaten erhalten.

Um in Wasser gute Suspensionen oder Emulsionen zu erreichen, werden weiterhin Netzmittel aus der obengenannten Reihe zugesetzt.

Bei herbiziden Mitteln können die Konzentrationen der Wirkstoffe in den handelsüblichen Formulierungen verschieden sein. In benetzbaren Pulvern variiert die Wirkstoffkonzentration z. B. zwischen etwa 10% und 80%, der Rest besteht aus den oben angegebenen Formulierungszusätzen. Bei emulgierbaren Konzentraten ist die Wirkstoffkonzentration etwa 10% bis 60%.

Zur Anwendung werden die handelsüblichen Konzentrate gegebenenfalls in üblicher Weise verdünnt, z. B. bei benetzbaren Pulvern und emulgierbaren Konzentraten mittels Wasser.

Versprühbare Lösungen werden vor der Anwendung nicht mehr mit weiteren inerten Stoffen verdünnt. Mit den äußeren Bedingungen wie Temperatur, Feuchtigkeit u.a. variiert die erforderliche Aufwandmenge. Sie kann in weiten Grenzen schwanken, z. B. zwischen 0,1 kg/ha und 10 kg/ha Aktivsubstanz, liegt jedoch vorzugsweise zwischen 0,3 und 3 kg/ha.

Folgende Beispiele dienen der weiteren Erläuterung der Erfindung:

## A. CHEMISCHE BEISPIELE

### Beispiel 1

N-DL-Phosphinothricyl-DL-leucinmethylester

23,6 g (0,075 mol) Cbo-DL-Phosphinothricin und 15,0 g (0,0825 mol) DL-Leucinmethylesterhydrochlorid werden mit 7,6 g (0,075 mol) Triethylamin in 150 ml Tetrahydrofuran gelöst. Bei 0°C tropft man eine Lösung von 19,5 g (0,09 mol) Dicyclohexylcarbodiimid in 30 ml Tetrahydrofuran zu. Man rührt 15 Stunden bei 0°C nach, zersetzt überschüssiges Dicyclohexylcarbodiimid mit 2 ml Eisessig, saugt den ausgefallenen Feststoff ab und engt das Filtrat im Vakuum ein. Der Rückstand wird in Essigsäureethylester aufgenommen und mit 0,5 N Salzsäure und Wasser gewaschen. Nach Trocknen der organischen Phase mit $Na_2SO_4$ und Einengen im Vakuum hinterbleiben 29,9 g roher N-Cbo-DL-Phosphinothricyl-DL-leucinmethylester. Die Abspaltung der Cbo-Schutzgruppe erfolgt durch Lösen in 200 ml Methanol, Zugeben von 4,5 ml Eisessig und 3 g Palladium auf Aktivkohle (10 %) und Einleiten eines $H_2$-Stromes in die auf 40°C erwärmte Lösung bis keine $CO_2$-Entwicklung mehr zu beobachten ist. Man filtriert den Katalysator ab und dampft im Vakuum ein. Verreiben des Rückstandes mit Aceton, Absaugen und Trocknen liefert 15,0 g (Ausbeute über beide Stufen 65 %) N-DL-Phosphinothricyl-DL-leucinmethylester vom Schmp. 153-155°C.

0232500

Beispiel 2

N-DL-Phosphinothricyl-DL-leucin

10,0 g (0,032 mol) N-DL-Phosphinothricyl-DL-leucinmethylester werden in 60 ml konz. Salzsäure 2 Stunden auf 50°C erwärmt. Man dampft im Vakuum zur Trockne ein und löst den Rückstand in 100 ml absolutem Ethanol. Nach Zugabe von 10 ml Propylenoxid rührt man 5 Stunden bei Raumtemperatur, filtriert ab und trocknet. Man erhält 6,5 g (69 %) N-DL-Phosphinothricyl-DL-leucin vom Schmp. 206°C.

Beispiel 3

N-DL-Phosphinothricyl-L-phenylalanyl-DL-alaninhydrochlorid

7,0 g (0,017 mol) N-DL-Phosphinothricyl-L-phenylalanyl-DL-alaninmethylester werden in 80 ml konz. Salzsäure 2 Stunden auf 40°C erwärmt. Man dampft im Vakuum ein, trocknet und erhält 6,6 g (89 %) N-DL-Phosphinothricyl-L-phenylalanyl-DL-alaninhydrochlorid vom Schmp. 154-155°C (Zers.).

Die nachfolgenden in Tabelle 1 aufgeführten Verbindungen der allgemeinen Formel I können in analoger Weise erhalten werden.

TABELLE 1*

H-AminosäureI-(AminosäureII)$_n$-AminosäureIII-OR · (HX)$_m$

| Bsp.-Nr. | Amino-säure I | Amino-säure II | Amino-säure III | R | X | m | n | Fp. [°C] |
|---|---|---|---|---|---|---|---|---|
| 4 | L-Ptc | - | Gly | $CH_3$ | - | 0 | 0 | |
| 5 | L-Ptc | - | Gly | H | Cl | 1 | 0 | |
| 6 | L-Ptc | - | Gly | Na | - | 0 | 0 | |
| 7 | DL-Ptc | - | Gly | $CH_3$ | - | 0 | 0 | 255-260 Z. |
| 8 | DL-Ptc | - | Gly | H | Cl | 1 | 0 | |
| 9 | L-Ptc | - | L-Ala | $CH_3$ | - | 0 | 0 | |
| 10 | L-Ptc | - | L-Ala | H | Cl | 1 | 0 | |
| 11 | L-Ptc | - | DL-Ala | $CH_3$ | - | 0 | 0 | |
| 12 | L-Ptc | - | DL-Ala | H | Cl | 1 | 0 | |
| 13 | DL-Ptc | - | L-Ala | $CH_3$ | - | 0 | 0 | |
| 14 | DL-Ptc | - | L-Ala | H | Cl | 1 | 0 | |
| 15 | DL-Ptc | - | DL-Ala | H | Cl | 1 | 0 | |
| 16 | L-Ptc | - | β-Ala | H | - | 0 | 0 | |
| 17 | DL-Ptc | - | β-Ala | H | Cl | 1 | 0 | Glas |
| 18 | DL-Ptc | - | β-Ala | H | - | 0 | 0 | |
| 19 | L-Ptc | - | L-Val | H | - | 0 | 0 | |
| 20 | L-Ptc | - | DL-Val | H | - | 0 | 0 | |
| 21 | DL-Ptc | - | L-Val | H | - | 0 | 0 | |

./.

Forts. Tabelle 1

| Bsp.-Nr. | Amino-säure I | Amino-säure II | Amino-säure III | R | X | m | n | Fp. [°C] |
|---|---|---|---|---|---|---|---|---|
| 22 | DL-Ptc | - | DL-Val | H | - | 0 | 0 | 218 |
| 23 | DL-Ptc | - | DL-Val | $CH_3$ | - | 0 | 0 | 165-166 |
| 24 | L-Ptc | - | L-Leu | H | - | 0 | 0 | |
| 25 | L-Ptc | - | DL-Leu | H | - | 0 | 0 | |
| 26 | DL-Ptc | - | L-Leu | H | - | 0 | 0 | |
| 27 | DL-Ptc | - | DL-Leu | H | Cl | 1 | 0 | |
| 28 | DL-Ptc | - | L-Leu | $CH_3$ | - | 0 | 0 | |
| 29 | L-Ptc | - | L-Ile | H | - | 0 | 0 | |
| 30 | L-Ptc | - | DL-Ile | H | - | 0 | 0 | |
| 31 | DL-Ptc | - | L-Ile | H | - | 0 | 0 | 217 |
| 32 | DL-Ptc | - | DL-Ile | H | - | 0 | 0 | |
| 33 | L-Ptc | - | L-Phg | H | - | 0 | 0 | |
| 34 | L-Ptc | - | DL-Phg | H | - | 0 | 0 | |
| 35 | DL-Ptc | - | L-Phg | H | - | 0 | 0 | |
| 36 | DL-Ptc | - | DL-Phg | H | - | 0 | 0 | 120 Zers. |
| 37 | DL-Ptc | - | DL-Phg | $CH_3$ | - | 0 | 0 | 182-184 Z. |
| 38 | D-Ptc | - | L-Phe | H | - | 0 | 0 | |
| 39 | L-Ptc | - | DL-Phe | H | - | 0 | 0 | |
| 40 | DL-Ptc | - | L-Phe | H | - | 0 | 0 | 215-219 Z. |
| 41 | DL-Ptc | - | DL-Phe | H | - | 0 | 0 | Glas |
| 42 | DL-Ptc | - | L-Phe | $CH_3$ | - | 0 | 0 | 210 |
| 43 | DL-Ptc | - | DL-Phe | $CH_3$ | Cl | 1 | 0 | 148-150 Z. |
| 44 | DL-Ptc | - | D-Phe | $CH_3$ | Cl | 1 | 0 | 120-123 Z. |
| 45 | DL-Ptc | - | D-Phe | H | Cl | 1 | 0 | 135 Z. |

0232500

Forts. Tabelle 1

| Bsp.-Nr. | Aminosäure I | Aminosäure II | Aminosäure III | R | X | m | n | Fp. [°C] |
|---|---|---|---|---|---|---|---|---|
| 46 | L-Ptc | - | L-Ser | H | - | 0 | 0 | |
| 47 | L-Ptc | - | DL-Ser | H | - | 0 | 0 | |
| 48 | DL-Ptc | - | L-Ser | H | - | 0 | 0 | |
| 49 | DL-Ptc | - | DL-Ser | H | - | 0 | 0 | |
| 50 | L-Ptc | - | L-Thr | H | - | 0 | 0 | |
| 51 | L-Ptc | - | DL-Thr | H | - | 0 | 0 | |
| 52 | DL-Ptc | - | L-Thr | H | - | 0 | 0 | |
| 53 | DL-Ptc | - | DL-Thr | H | - | 0 | 0 | |
| 54 | L-Ptc | - | L-Met | H | - | 0 | 0 | |
| 55 | L-Ptc | - | DL-Met | H | - | 0 | 0 | |
| 56 | DL-Ptc | - | L-Met | H | - | 0 | 0 | |
| 57 | DL-Ptc | - | DL-Met | H | - | 0 | 0 | |
| 58 | L-Ptc | - | L-Tyr | H | - | 0 | 0 | |
| 59 | L-Ptc | - | DL-Tyr | H | - | 0 | 0 | |
| 60 | DL-Ptc | - | L-Tyr | H | - | 0 | 0 | |
| 61 | DL-Ptc | - | DL-Tyr | H | - | 0 | 0 | |
| 62 | L-Ptc | - | L-Glu | H | - | 0 | 0 | |
| 63 | L-Ptc | - | DL-Glu | H | - | 0 | 0 | |
| 64 | DL-Ptc | - | L-Glu | H | - | 0 | 0 | |
| 65 | DL-Ptc | - | DL-Glu | H | - | 0 | 0 | |
| 66 | L-Ptc | - | L-Gln | H | - | 0 | 0 | |

./.

Forts. Tabelle 1

| Bsp.-Nr. | Aminosäure I | Aminosäure II | Aminosäure III | R | X | m | n | Fp. [°C] |
|---|---|---|---|---|---|---|---|---|
| 67 | L-Ptc | - | DL-Gln | H | - | 0 | 0 | |
| 68 | DL-Ptc | - | L-Gln | H | - | 0 | 0 | |
| 69 | DL-Ptc | - | DL-Gln | H | - | 0 | 0 | |
| 70 | L-Ptc | - | L-Asp | H | - | 0 | 0 | |
| 71 | L-Ptc | - | DL-Asp | H | - | 0 | 0 | |
| 72 | DL-Ptc | - | L-Asp | H | - | 0 | 0 | |
| 73 | DL-Ptc | - | DL-Asp | H | - | 0 | 0 | |
| 74 | L-Ptc | - | L-Asn | H | - | 0 | 0 | |
| 75 | L-Ptc | - | DL-Asn | H | - | 0 | 0 | |
| 76 | DL-Ptc | - | L-Asn | H | - | 0 | 0 | |
| 77 | DL-Ptc | - | DL-Asn | H | - | 0 | 0 | |
| 78 | L-Ptc | - | L-Lys | H | - | 0 | 0 | |
| 79 | L-Ptc | - | DL-Lys | H | - | 0 | 0 | |
| 80 | DL-Ptc | - | L-Lys | H | - | 0 | 0 | 187-190 Z. |
| 81 | DL-Ptc | - | DL-Lys | H | - | 0 | 0 | |
| 82 | L-Ptc | - | L-Nle | H | - | 0 | 0 | |
| 83 | L-Ptc | - | DL-Nle | H | - | 0 | 0 | |
| 84 | DL-Ptc | - | L-Nle | H | - | 0 | 0 | |
| 85 | DL-Ptc | - | DL-Nle | H | - | 0 | 0 | |
| 86 | L-Ptc | - | L-Nva | H | - | 0 | 0 | |
| 87 | L-Ptc | - | DL-Nva | H | - | 0 | 0 | |
| 88 | DL-Ptc | - | L-Nva | H | - | 0 | 0 | |

./.

Forts. Tabelle I

| Bsp.-Nr. | Aminosäure I | Aminosäure II | Aminosäure III | R | X | m | n | Fp. [°C] |
|---|---|---|---|---|---|---|---|---|
| 89 | DL-Ptc | - | DL-Nva | H | - | 0 | 0 | 260 Zers. |
| 90 | L-Ptc | - | L-Cys | H | - | 0 | 0 | |
| 91 | L-Ptc | - | DL-Cys | H | - | 0 | 0 | |
| 92 | DL-Ptc | - | L-Cys | H | - | 0 | 0 | |
| 93 | DL-Ptc | - | DL-Cys | H | - | 0 | 0 | |
| 94 | L-Ptc | - | L-Abu | H | - | 0 | 0 | |
| 95 | L-Ptc | - | DL-Abu | H | - | 0 | 0 | |
| 96 | DL-Ptc | - | L-Abu | H | - | 0 | 0 | |
| 97 | DL-Ptc | - | DL-Abu | H | - | 0 | 0 | |
| 98 | L-Ptc | - | $\gamma$-Abu | H | - | 0 | 0 | |
| 99 | DL-Ptc | - | $\gamma$-Abu | H | - | 0 | 0 | |
| 100 | DL-Ptc | - | $\gamma$-Abu | $CH_3$ | - | 0 | 0 | 238 |
| 101 | L-Ptc | - | DL-Phg(4Cl) | H | - | 0 | 0 | |
| 102 | DL-Ptc | - | DL-Phg(4Cl) | H | - | 0 | 0 | |
| 103 | L-Ptc | - | DL-Phg(2Cl4$CF_3$) | H | - | 0 | 0 | |
| 104 | DL-Ptc | - | DL-Phg(2Cl4$CF_3$) | H | - | 0 | 0 | |
| 105 | L-Ptc | - | DL-Phe(4Cl) | H | - | 0 | 0 | |
| 106 | DL-Ptc | - | DL-Phe(4Cl) | H | - | 0 | 0 | |
| 107 | L-Ptc | - | DL-Phe (4$NO_2$) | H | - | 0 | 0 | |
| 108 | DL-Ptc | - | DL-Phe(4$NO_2$) | H | - | 0 | 0 | |
| 109 | L-Ptc | - | DL-Val($\alpha$Me) | H | - | 0 | 0 | |
| 110 | DL-Ptc | - | DL-Val($\alpha$Me) | H | - | 0 | 0 | |

./.

Forts. Tabelle 1

| Bsp.-Nr. | Amino-säure I | Amino-säure II | Amino-säure III | R | X | m | n | Fp. [°C] |
|---|---|---|---|---|---|---|---|---|
| 111 | DL-Ptc | Gly | DL-Ala | $CH_3$ | - | 0 | 1 | 145-147 Z. |
| 112 | L-Ptc | Gly | β-Ala | H | - | 0 | 1 | |
| 113 | DL-Ptc | Gly | β-Ala | H | - | 0 | 1 | |
| 114 | L-Ptc | Gly | L-Leu | H | - | 0 | 1 | |
| 115 | DL-Ptc | Gly | DL-Leu | H | - | 0 | 1 | |
| 116 | L-Ptc | Gly | L-Ile | H | - | 0 | 1 | |
| 117 | DL-Ptc | Gly | L-Ile | H | - | 0 | 1 | |
| 118 | L-Ptc | Gly | L-Phg | H | - | 0 | 1 | |
| 119 | DL-Ptc | Gly | DL-Phg | H | - | 0 | 1 | |
| 120 | L-Ptc | Gly | L-Phe | H | - | 0 | 1 | |
| 121 | DL-Ptc | Gly | DL-Phe | H | - | 0 | 1 | |
| 122 | L-Ptc | Gly | L-Met | H | - | 0 | 1 | |
| 123 | DL-Ptc | Gly | DL-Met | H | - | 0 | 1 | |
| 124 | L-Ptc | Gly | L-Nle | H | - | 0 | 1 | |
| 125 | DL-Ptc | Gly | DL-Nle | H | - | 0 | 1 | |
| 126 | L-Ptc | Gly | L-Nva | H | - | 0 | 1 | |
| 127 | DL-Ptc | Gly | DL-Nva | H | - | 0 | 1 | |

Forts. Tabelle 1

| Bsp.-Nr. | Aminosäure I | Aminosäure II | Aminosäure III | R | X | m | n | Fp. (°C) |
|---|---|---|---|---|---|---|---|---|
| 128 | DL-Ptc | DL-Ala | DL-Val | $CH_3$ | - | 0 | 1 | 186-188 Z. |
| 129 | L-Ptc | L-Ala | DL-Leu | $CH_3$ | - | 0 | 1 | |
| 130 | DL-Ptc | DL-Ala | DL-Leu | $C_2H_5$ | - | 0 | 1 | |
| 131 | L-Ptc | L-Ala | L-Ile | H | - | 0 | 1 | |
| 132 | DL-Ptc | DL-Ala | DL-Ile | H | - | 0 | 1 | |
| 133 | L-Ptc | L-Ala | L-Phg | H | - | 0 | 1 | |
| 134 | DL-Ptc | DL-Ala | DL-Phg | H | - | 0 | 1 | |
| 135 | DL-Ptc | L-Ala | DL-Phg | $CH_3$ | - | 0 | 1 | 142-145 Z. |
| 136 | DL-Ptc | DL-Ala | DL-Phg | H | Cl | 0 | 1 | 115-118 Z. |
| 137 | L-Ptc | L-Ala | L-Phe | H | - | 0 | 1 | |
| 138 | DL-Ptc | DL-Ala | DL-Phe | H | - | 0 | 1 | |
| 139 | DL-Ptc | DL-Ala | L-Phe | $CH_3$ | - | 0 | 1 | 131-134 Z. |
| 140 | DL-Ptc | DL-Ala | L-Phe | H | Cl | 1 | 1 | 148-151 Z. |
| 141 | L-Ptc | L-Ala | L-Met | H | - | 0 | 1 | |
| 142 | DL-Ptc | DL-Ala | DL-Met | H | - | 0 | 1 | |
| 143 | L-Ptc | L-Ala | L-Nle | H | - | 0 | 1 | |
| 144 | DL-Ptc | DL-Ala | DL-Nle | H | - | 0 | 1 | |
| 145 | L-Ptc | L-Ala | L-Nva | H | - | 0 | 1 | |
| 146 | DL-Ptc | DL-Ala | DL-Nva | H | - | 0 | 1 | |

Forts. Tabelle 1

| Bsp.-Nr. | Aminosäure I | Aminosäure II | Aminosäure III | R | X | m | n | Fp. [°C] |
|---|---|---|---|---|---|---|---|---|
| 147 | DL-Ptc | DL-Ala | γ-Abu | H | - | 0 | 1 | |
| 148 | L-Ptc | L-Ala | L-Thr | H | - | 0 | 1 | |
| 149 | L-Ptc | L-Ala | L-Tyr | H | - | 0 | 1 | |
| 150 | L-Ptc | L-Ala | L-Glu | H | - | 0 | 1 | |
| 151 | L-Ptc | L-Ala | L-Gln | H | - | 0 | 1 | |
| 152 | L-Ptc | L-Ala | L-Asp | H | - | 0 | 1 | |
| 153 | L-Ptc | L-Ala | L-Asn | H | - | 0 | 1 | |
| 154 | L-Ptc | L-Ala | L-Lys | H | - | 0 | 1 | |
| 155 | L-Ptc | L-Ala | L-Cys | H | - | 0 | 1 | |
| 156 | DL-Ptc | DL-Ala | DL-Phg(4Cl) | H | - | 0 | 1 | |
| 157 | DL-Ptc | DL-Ala | DL-Phe(4Cl) | H | - | 0 | 1 | |
| 158 | DL-Ptc | DL-Ala | DL-Val(αMe) | H | - | 0 | 1 | |
| 159 | DL-Ptc | DL-Ala | Ala(αMe) | H | - | 0 | 1 | |
| 160 | L-Ptc | β-Ala | β-Ala | H | - | 0 | 1 | |
| 161 | L-Ptc | β-Ala | L-Val | H | - | 0 | 1 | |
| 162 | L-Ptc | β-Ala | L-Abu | H | - | 0 | 1 | |
| 163 | L-Ptc | β-Ala | L-Phg | H | - | 0 | 1 | |
| 164 | L-Ptc | β-Ala | L-Phe | H | - | 0 | 1 | |
| 165 | L-Ptc | β-Ala | L-Leu | H | - | 0 | 1 | |
| 166 | L-Ptc | L-Val | β-Ala | H | - | 0 | 1 | |

Forts. Tabelle 1

| Bsp.-Nr. | Amino-säure I | Amino-säure II | Amino-säure III | R | X | m | n | Fp. (°C) |
|---|---|---|---|---|---|---|---|---|
| 167 | L-Ptc | L-Val | L-Phg | H | - | 0 | 1 | |
| 168 | L-Ptc | L-Val | L-Phe | H | - | 0 | 1 | |
| 169 | L-Ptc | L-Val | L-Leu | H | - | 0 | 1 | |
| 170 | L-Ptc | L-Abu | β-Ala | H | - | 0 | 1 | |
| 171 | L-Ptc | L-Abu | L-Phg | H | - | 0 | 1 | |
| 172 | L-Ptc | L-Abu | L-Phe | H | - | 0 | 1 | |
| 173 | L-Ptc | L-Abu | L-Leu | H | - | 0 | 1 | |
| 174 | L-Ptc | L-Phg | β-Ala | H | - | 0 | 1 | |
| 175 | L-Ptc | L-Phg | L-Val | H | - | 0 | 1 | |
| 176 | L-Ptc | L-Phg | L-Abu | H | - | 0 | 1 | |
| 177 | L-Ptc | L-Phg | L-Phg | H | - | 0 | 1 | |
| 178 | L-Ptc | L-Phg | L-Phe | H | - | 0 | 1 | |
| 179 | L-Ptc | L-Phg | L-Leu | H | - | 0 | 1 | |
| 180 | L-Ptc | L-Phe | β-Ala | H | - | 0 | 1 | |
| 181 | L-Ptc | L-Phe | L-Val | H | - | 0 | 1 | |
| 182 | L-Ptc | L-Phe | L-Abu | H | - | 0 | 1 | |
| 183 | L-Ptc | L-Phe | L-Phg | H | - | 0 | 1 | |
| 184 | L-Ptc | L-Phe | L-Phe | H | - | 0 | 1 | |
| 185 | L-Ptc | L-Phe | L-Leu | H | - | 0 | 1 | |
| 186 | L-Ptc | L-Leu | β-Ala | H | - | 0 | 1 | |
| 187 | L-Ptc | L-Leu | L-Val | H | - | 0 | 1 | |

Forts. Tabelle 1

| Bsp.-Nr. | Aminosäure I | Aminosäure II | Aminosäure III | R | X | m | n | Fp. (°C) |
|---|---|---|---|---|---|---|---|---|
| 188 | L-Ptc | L-Leu | L-Abu | H | - | 0 | 1 | |
| 189 | L-Ptc | L-Leu | L-Phg | H | - | 0 | 1 | |
| 190 | L-Ptc | L-Leu | L-Phe | H | - | 0 | 1 | |
| 191 | L-Ptc | L-Leu | L-Leu | H | - | 0 | 1 | |
| 192 | L-Ptc | β-Ala | Gly | H | - | 0 | 1 | |
| 193 | DL-Ptc | β-Ala | Gly | H | - | 0 | 1 | |
| 194 | L-Ptc | L-Val | Gly | H | - | 0 | 1 | |
| 195 | DL-Ptc | DL-Val | Gly | H | - | 0 | 1 | |
| 196 | L-Ptc | L-Leu | Gly | H | - | 0 | 1 | |
| 197 | DL-Ptc | DL-Leu | Gly | H | - | 0 | 1 | |
| 198 | L-Ptc | L-Ile | Gly | H | - | 0 | 1 | |
| 199 | DL-Ptc | L-Ile | Gly | H | - | 0 | 1 | |
| 200 | L-Ptc | L-Phg | Gly | H | - | 0 | 1 | |
| 201 | DL-Ptc | DL-Phg | Gly | H | - | 0 | 1 | |
| 202 | L-Ptc | L-Phe | Gly | H | - | 0 | 1 | |
| 203 | DL-Ptc | DL-Phe | Gly | H | - | 0 | 1 | |
| 204 | L-Ptc | L-Met | Gly | H | - | 0 | 1 | |
| 205 | DL-Ptc | DL-Met | Gly | H | - | 0 | 1 | |
| 206 | L-Ptc | L-Nle | Gly | H | - | 0 | 1 | |
| 207 | DL-Ptc | DL-Nle | Gly | H | - | 0 | 1 | |
| 208 | L-Ptc | L-Nva | Gly | H | - | 0 | 1 | |
| 209 | DL-Ptc | DL-Nva | Gly | H | - | 0 | 1 | |
| 210 | L-Ptc | β-Ala | L-Ala | H | - | 0 | 1 | |
| 211 | DL-Ptc | β-Ala | DL-Ala | H | - | 0 | 1 | |

Forts. Tabelle 1

| Bsp.-Nr. | Amino-säure I | Amino-säure II | Amino-säure III | R | X | m | n | Fp. [°C] |
|---|---|---|---|---|---|---|---|---|
| 212 | DL-Ptc | DL-Val | DL-Ala | $CH_3$ | - | 0 | 1 | Glas |
| 213 | L-Ptc | L-Ile | L-Ala | H | - | 0 | 1 | |
| 214 | DL-Ptc | DL-Ile | DL-Ala | H | - | 0 | 1 | |
| 215 | L-Ptc | L-Leu | L-Ala | H | - | 0 | 1 | |
| 216 | DL-Ptc | DL-Leu | DL-Ala | H | - | 0 | 1 | |
| 217 | L-Ptc | L-Phg | L-Ala | H | - | 0 | 1 | |
| 218 | DL-Ptc | DL-Phg | DL-Ala | H | - | 0 | 1 | |
| 219 | DL-Ptc | DL-Phg | DL-Ala | $CH_3$ | - | 0 | 1 | 169-172 Z. |
| 220 | DL-Ptc | DL-Phg | DL-Ala | H | Cl | 1 | 1 | 155-158 Z. |
| 221 | L-Ptc | L-Phe | L-Ala | H | - | 0 | 1 | |
| 222 | DL-Ptc | DL-Phe | DL-Ala | H | - | 0 | 1 | |
| 223 | DL-Ptc | L-Phe | DL-Ala | $CH_3$ | - | 0 | 1 | 171-174 Z. |
| 224 | DL-Ptc | DL-Phe | DL-Ala | H | Cl | 1 | 1 | |
| 225 | L-Ptc | L-Met | L-Ala | H | - | 0 | 1 | |
| 226 | DL-Ptc | DL-Met | DL-Ala | H | - | 0 | 1 | |
| 227 | L-Ptc | L-Nle | L-Ala | H | - | 0 | 1 | |
| 228 | DL-Ptc | DL-Nle | DL-Ala | H | - | 0 | 1 | |
| 229 | L-Ptc | L-Nva | L-Ala | H | - | 0 | 1 | |
| 230 | DL-Ptc | DL-Nva | DL-Ala | H | - | 0 | 1 | |
| 231 | DL-Ptc | γ-Abu | DL-Ala | H | - | 0 | 1 | |
| 232 | L-Ptc | L-Thr | L-Ala | H | - | 0 | 1 | |
| 233 | L-Ptc | L-Tyr | L-Ala | H | - | 0 | 1 | |

Forts. Tabelle 1

| Bsp.-Nr. | Aminosäure I | Aminosäure II | Aminosäure III | R | X | m | n | Fp. [°C] |
|---|---|---|---|---|---|---|---|---|
| 234 | L-Ptc | L-Glu | L-Ala | H | - | 0 | 1 | |
| 235 | L-Ptc | L-Gln | L-Ala | H | - | 0 | 1 | |
| 236 | L-Ptc | L-Asp | L-Ala | H | - | 0 | 1 | |
| 237 | L-Ptc | L-Asn | L-Ala | H | - | 0 | 1 | |
| 238 | L-Ptc | L-Lys | L-Ala | H | - | 0 | 1 | |
| 239 | L-Ptc | L-Cys | L-Ala | H | - | 0 | 1 | |
| 240 | DL-Ptc | DL-Phg(4Cl) | DL-Ala | H | - | 0 | 1 | |
| 241 | DL-Ptc | DL-Phe(4Cl) | DL-Ala | H | - | 0 | 1 | |
| 242 | DL-Ptc | DL-Val(αMe) | DL-Ala | H | - | 0 | 1 | |
| 243 | DL-PTc | Ala(αMe) | DL-Ala | H | - | 0 | 1 | |
| 244 | DL-Ptc | Ala(αMe) | L-Ala | H | - | 0 | 1 | |

* Die einzelnen Aminosäuren werden durch Abkürzungen bezeichnet, die in der Peptidchemie allgemein gebräuchlich sind (Houben-Weyl, Bd. 15/1, S.2 ff). Phosphinothricin erhält die Abkürzung "Ptc". In Großbuchstaben vor der jeweiligen Aminosäure wird die Stereochemie der vorhandenen Asymmetriezentren angegeben. Z= Zersetzungspunkt.

./.

## B. FORMULIERUNGSBEISPIELE

### Beispiel 1

Eine 20 %ige wäßrige Lösung wird erhalten aus:

20 Gew.-% Wirkstoff
10 Gew.-% Nonylphenol · 10 AeO
20 Gew.-% Ethylenglykol
50 Gew.-% Wasser

### Beispiel 2

Ein in Wasser leicht dispergierbares, benetzbares Pulver wird erhalten, indem man

25 Gew.-% Wirkstoff
64 Gew.-% kaolinhaltiges Quarz als Inertstoff
10 Gew.-% ligninsulfonsaures Kalium
und
1 Gew.-% oleylmethyltaurinsaures Natrium als Netz- und Dispergiermittel

mischt und in einer Stiftmühle mahlt.

./.

## C. BIOLOGISCHE BEISPIELE

Die Schädigung der Unkrautpflanzen bzw. die Kulturpflanzenverträglichkeit wurde gemäß einem Schlüssel bonitiert, in dem die Wirksamkeit durch Wertzahlen von 0-5 ausgedrückt ist. Dabei bedeutet:

0 = ohne Wirkung bzw. Schaden
1 = 0 - 20% Wirkung bzw. Schaden
2 =20 - 40% Wirkung bzw. Schaden
3 =40 - 60% Wirkung bzw. Schaden
4 =60 - 80% Wirkung bzw. Schaden
5 =80 -100% Wirkung bzw. Schaden

### 1. Unkrautwirkung im Nachauflauf

Samen bzw. Rhizomstücke von mono- und dikotylen Unkräutern wurden in Plastiktöpfen (Ø = 9 cm) in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver, als Emulsionskonzentrate bzw. als wässrige Lösungen formulierten erfindungsgemäßen Verbindungen wurden in verschiedenen Dosierungen mit einer Wasseraufwandmenge von umgerechnet 600 l/ha auf die grünen Pflanzenteile gesprüht und nach ca. 3-4 Wochen Standzeit der Versuchspflanzen im Gewächshaus unter optimalen Wachstumsbedingungen (Temperatur 23 plus/minus 1°C, relative Luftfeuchte 60-80%) die Wirkung der Präparate optisch im Vergleich zu unbehandelten Kontrollen bonitiert. Die erfindungsgemäßen

Mittel weisen im Nachauflauf eine gute herbizide Wirksamkeit gegen ein breites Spektrum wirtschaftlich wichtiger Ungräser und Unkräuter auf.

TABELLE 2

Herbizide Wirkung der erfindungsgemäßen Verbindungen. Nachauflaufverfahren

| Produkt Bsp.-Nr. | Dosis kg a.i./ha | herbizide Wirkung AVF | SAL | ECG | SIA | CRS | STM |
|---|---|---|---|---|---|---|---|
| 23 | 2,4 | 3 | 5 | 4 | 5 | 5 | 5 |
| | 0,6 | 1 | 4 | 3 | 4 | 5 | 4 |
| 1 | 2,4 | 1 | 5 | 3 | 3 | 3 | 3 |
| | 0,6 | 1 | 3 | 1 | 5 | 5 | 4 |
| 7 | 2,4 | 4 | 5 | 5 | 5 | 5 | 5 |
| | 0,6 | 2 | 5 | 5 | 5 | 5 | 5 |
| 42 | 2,4 | 5 | 5 | 5 | 5 | 5 | 5 |
| | 0,6 | 3 | 5 | 5 | 5 | 5 | 4 |
| 22 | 2,4 | 4 | 5 | 5 | 5 | 5 | 5 |
| | 0,6 | 1 | 3 | 2 | 3 | 5 | 3 |
| 100 | 2,4 | 3 | 5 | 5 | 5 | 5 | 5 |
| | 0,6 | 2 | 4 | 3 | 5 | 5 | 4 |
| 31 | 2,4 | 2 | 4 | 5 | 5 | 5 | 5 |
| | 0,6 | 1 | 2 | 2 | 4 | 5 | 5 |
| 2 | 2,4 | 3 | 5 | 5 | 5 | 5 | 5 |
| | 0,6 | 2 | 4 | 3 | 5 | 5 | 4 |
| 37 | 2,4 | 4 | 5 | 5 | 5 | 5 | 5 |
| | 0,6 | 2 | 5 | 4 | 5 | 5 | 5 |

./.

Forts. Tabelle 2

| Produkt Bsp.-Nr. | Dosis kg a.i./ha | AVF | SAL | ECG | SIA | CRS | STM |
|---|---|---|---|---|---|---|---|
| | | herbizide Wirkung | | | | | |
| 40 | 2,4 | 5 | 5 | 5 | 5 | 5 | 5 |
| | 0,6 | 4 | 5 | 5 | 5 | 5 | 5 |
| 89 | 2,4 | 4 | 5 | 5 | 5 | 5 | 5 |
| | 0,6 | 1 | 5 | 4 | 4 | 5 | 4 |
| 80 | 2,4 | 3 | 5 | 4 | 4 | 4 | 4 |
| | 0,6 | 1 | 4 | 1 | 1 | 2 | 3 |
| 36 | 2,4 | - | - | 5 | 5 | 5 | 5 |
| | 0,6 | - | - | - | - | - | - |
| 17 | 2,4 | - | - | 5 | 5 | 5 | 5 |
| | 0,6 | - | - | - | - | - | - |
| 128 | 2,4 | - | - | 5 | 5 | 5 | 5 |
| | 0,6 | - | - | - | - | - | - |

Abkürzungen:

AVF= Avena fatua (Flughafer)

SAL= Setaria lutescens (Borstenhirse)

ECG= Echinochloa crus-galli (Hühnerhirse)

SIA= Sinapis alba (weißer Senf)

CRS= Chrysanthenum segetum (Saatwucherblume)

STM= Stellaria media (Sternmiere)

0232500
HCE 85/F 280

Patentansprüche:

1. Verbindungen der allgemeinen Formel I,

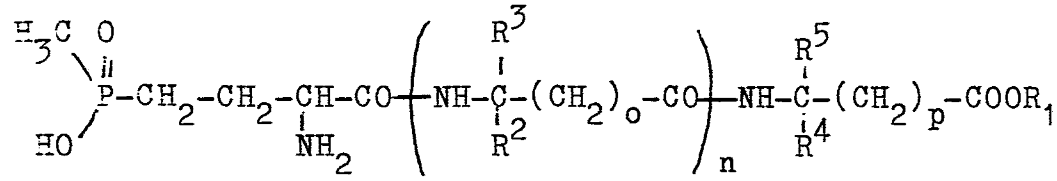

(I)

worin

$R^1$ Wasserstoff oder $(C_1-C_{12})$-Alkyl,

$R^2$ und $R^4$ unabhängig voneinander Wasserstoff, $(C_1-C_{12})$-Alkyl, $(C_3-C_8)$-Cycloalkyl, Phenyl oder Phenyl$(C_1-C_2)$-alkyl, wobei die genannten Gruppen ihrerseits durch Halogen, $NH_2$, OH, SH, $NO_2$, $CF_3$ $(C_1-C_4)$-Alkoxy, $(C_1-C_4)$-Alkylthio, $(C_1-C_4)$-Alkoxycarbonyl, COOH oder $CONH_2$ substituiert sein können,

$R^3$ und $R^5$ Wasserstoff oder Methyl

n 0 oder 1, und

o und p unabhängig voneinander 0, 1, 2 oder 3 bedeuten, ausgenommen Verbindungen, in denen

a) n= 0, $R^4$= H oder $CH_3$ und $R^5$= H; oder
b) n= 1, $R^3$ und $R^5$= H sowie $R^2$ und $R^4$ H, $CH_3$, $C_2H_5$, $CH(CH_3)_2$ oder $CH_2OH$; oder
c) n= 1, $R^3$ und $R^5$= H, $R^2$= $CH_3$ und $R^4$= $CH_2CH(CH_3)_2$ bedeuten,

sowie deren Salze mit Basen bzw. Säuren.

2. Verbindungen der Formel I, worin

$R^1$ Wasserstoff oder $(C_1-C_8)$-Alkyl,

$R^2$ und $R^4$ unabhängig voneinander H, $(C_1-C_4)$Alkyl, $(C_3-C_4)$Aminoalkyl, Hydroxymethyl, Hydroxyethyl, Thiomethyl, Methylthioethyl, Carboxymethyl oder -ethyl, Carbamoylmethyl oder -ethyl, Carbo$(C_1-C_4)$-alkoxymethyl oder -ethyl, Phenyl, Benzyl oder Hydroxybenzyl,

$R^3$ und $R^5$ H oder $CH_3$,

n 0 oder 1, und

o und p unabhängig voneinander 0, 1 oder 2 bedeuten, (ausgenommen die Verbindungen gemäß Anspruch 1, Abschnitte a), b) und c)). sowie deren Salze.

3. Verfahren zur Herstellung von Verbindungen der Formel I, gemäß den Ansprüchen 1 oder 2, dadurch gekennzeichnet, daß man eine Verbindung der Formel II,

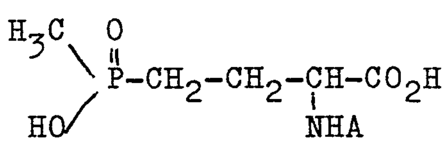

(II)

worin A eine Amino-Schutzgruppe bedeutet, mit dem Salz eines Aminosäure- bzw. Dipeptidderivats der Formel III,

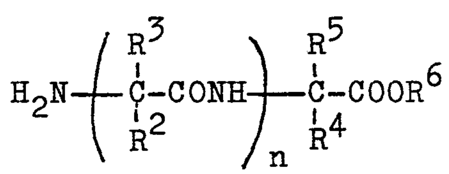

(III)

worin $R_6$ eine in der Peptidchemie gebräuchliche Carboxyl-Schutzgruppe wie Niederalkyl oder Benzyl ist, in Gegenwart einer Base und eines Dialkylcarbodiimids wie Dicyclohexylcarbodiimid (DCC) als Kondensationsmittel umsetzt und anschließend, soweit gewünscht, die vorhandenen Schutzgruppen abspaltet.

4. Mittel zur Unkrautbekämpfung, gekennzeichnet durch einen Gehalt an einer Verbindung der Formel I gemäß den Ansprüchen 1 oder 2.

5. Verwendung von Verbindungen der Formel I gemäß den Ansprüchen 1 oder 2 zur Bekämpfung unerwünschten Pflanzenwuchses.

6. Verfahren zur Bekämpfung unerwünschten Pflanzenwuchses, dadurch gekennzeichnet, daß man auf die zu bekämpfenden Pflanzen bzw. die zu behandelnde Fläche im Vorsaat-, Vorauflauf- oder Nachauflaufverfahren eine wirksame Menge einer Verbindung der Formel I gemäß den Ansprüchen 1 oder 2 aufbringt.

Patentansprüche Österreich:

1. Verfahren zur Herstellung von Verbindungen der Formel I

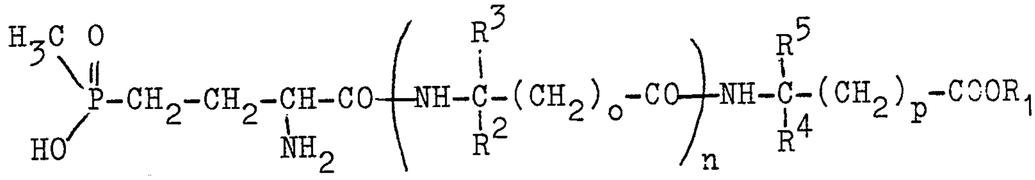

(I)

worin

$R^1$ Wasserstoff oder $(C_1-C_{12})$-Alkyl,

$R^2$ und $R^4$ unabhängig voneinander Wasserstoff, $(C_1-C_{12})$-Alkyl, $(C_3-C_8)$-Cycloalkyl, Phenyl oder Phenyl$(C_1-C_2)$-alkyl, wobei die genannten Gruppen ihrerseits durch Halogen, $NH_2$, OH, SH, $NO_2$, $CF_3$ $(C_1-C_4)$-Alkoxy, $(C_1-C_4)$-Alkylthio, $(C_1-C_4)$-Alkoxycarbonyl, COOH oder $CONH_2$ substituiert sein können,

$R^3$ und $R^5$ Wasserstoff oder Methyl

n 0 oder 1, und

o und p unabhängig voneinander 0, 1, 2 oder 3 bedeuten, ausgenommen Verbindungen, in denen

a) n= 0, $R^4$= H oder $CH_3$ und $R^5$= H; oder
b) n= 1, $R^3$ und $R^5$= H sowie $R^2$ und $R^4$ H, $CH_3$, $C_2H_5$, $CH(CH_3)_2$ oder $CH_2OH$; oder
c) n= 1, $R^3$ und $R^5$= H, $R^2$= $CH_3$ und $R^4$= $CH_2CH(CH_3)_2$ bedeuten,

sowie deren Salzen mit Basen bzw. Säuren, dadurch gekennzeichnet, daß man eine Verbindung der Formel II,

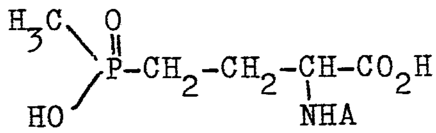 (II)

worin A eine Amino-Schutzgruppe bedeutet, mit dem Salz eines Aminosäure- bzw. Dipeptidderivats der Formel III,

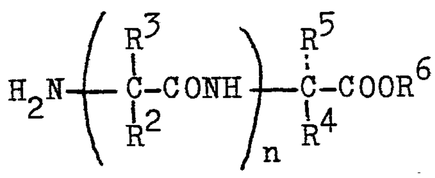 (III)

worin $R_6$ eine in der Peptidchemie gebräuchliche Carboxyl-Schutzgruppe wie Niederalkyl oder Benzyl ist, in Gegenwart einer Base und eines Dialkylcarbodiimids wie Dicyclohexylcarbodiimid (DCC) als Kondensationsmittel umsetzt und anschließend, soweit gewünscht, die vorhandenen Schutzgruppen abspaltet.

2. Verfahren zur Herstellung von Verbindungen der Formel I, worin

$R^1$ Wasserstoff oder $(C_1-C_8)$-Alkyl,

$R^2$ und $R^4$ unabhängig voneinander H, $(C_1-C_4)$Alkyl, $(C_3-C_4)$Aminoalkyl, Hydroxymethyl, Hydroxyethyl, Thiomethyl, Methylthioethyl, Carboxymethyl oder -ethyl, Carbamoylmethyl oder -ethyl, Carbo$(C_1-C_4)$-alkoxymethyl oder -ethyl, Phenyl, Benzyl oder Hydroxybenzyl,

$R^3$ und $R^5$ H oder $CH_3$,

n 0 oder 1, und

o und p unabhängig voneinander 0, 1 oder 2 bedeuten, (ausgenommen die vorstehend unter a), b) und c) aufgeführten Verbindungen sowie deren Salze.

3. Mittel zur Unkrautbekämpfung, gekennzeichnet durch einen Gehalt an einer Verbindung der Formel I gemäß den Ansprüchen 1 oder 2.

4. Verwendung von Verbindungen der Formel I gemäß den Ansprüchen 1 oder 2 zur Bekämpfung unerwünschten Pflanzenwuchses.

5. Verfahren zur Bekämpfung unerwünschten Pflanzenwuchses, dadurch gekennzeichnet, daß man auf die zu bekämpfenden Pflanzen bzw. die zu behandelnde Fläche im Vorsaat-, Vorauflauf- oder Nachauflaufverfahren eine wirksame Menge einer Verbindung der Formel I gemäß den Ansprüchen 1 oder 2 aufbringt.